# EUROPEAN PATENT SPECIFICATION

(11) **EP 3 089 957 B1**
(45) Date of publication and mention of the grant of the patent: **30.12.2020**
(21) Application number: 14709775.2
(22) Date of filing: 11.02.2014
(51) Int. Cl.: C07C 51/265, C07C 51/43, C07C 51/47

(54) **PURIFICATION OF AROMATIC CARBOXYLIC ACIDS**
REINIGUNG VON AROMATISCHEN CARBONSÄUREN
PURIFICATION D'ACIDES CARBOXYLIQUES AROMATIQUES

(30) Priority: 30.12.2013 US 201361921706 P
(43) Date of publication of application: 09.11.2016
(73) Proprietor: BP Corporation North America Inc., Houston, TX 77079 (US)
(72) Inventor: BARTOS, Thomas M., Naperville, IL 60563 (US)
(74) Representative: Hamer, Christopher K.
(86) International application number: PCT/US2014/015820
(87) International publication number: WO 2015/102654

(56) References cited:
- WO-A1-2006/102137
- WO-A2-2011/041151
- WO-A2-2011/100682
- GB-A- 785 045
- GB-A- 1 047 433
- US-A- 5 723 656

## Description

### CROSS-REFERENCE TO RELATED APPLICATIONS

This application claims the benefit of priority from U.S. Provisional Application No. 61/921,706, filed December 30, 2013.

### TECHNICAL FIELD

The present teachings relate generally to processes for manufacturing aromatic carboxylic acids, and in particular, to processes for preparing crude aromatic carboxylic acids for purification.

### BACKGROUND

Terephthalic acid (TA) and other aromatic carboxylic acids may be used in the manufacture of polyesters (e.g., via their reaction with ethylene glycol and/or higher alkylene glycols). Polyesters in turn may be used to make fibers, films, containers, bottles, other packaging materials, molded articles, and the like.

In commercial practice, aromatic carboxylic acids have been made by liquid phase oxidation of methyl-substituted benzene and naphthalene feedstocks in an aqueous acetic acid solvent. The positions of the methyl substituents correspond to the positions of carboxyl groups in the aromatic carboxylic acid product. Air or other sources of oxygen (e.g., typically in a gaseous state) have been used as oxidants in the presence, for example, of a bromine-promoted catalyst that contains cobalt and manganese. The oxidation is exothermic and yields aromatic carboxylic acid together with by-products, including partial or intermediate oxidation products of the aromatic feedstock, and acetic acid reaction products (e.g., methanol, methyl acetate, and methyl bromide). Water is also generated as a byproduct.

Pure forms of aromatic carboxylic acids are oftentimes desirable for the manufacture of polyesters to be used in important applications (e.g., fibers and bottles). Impurities in the acids (e.g., by-products generated from oxidation of aromatic feedstocks and, more generally, various carbonyl-substituted aromatic species) are thought to cause and/or correlate with color formation in polyesters made therefrom, which in turn leads to off-color in polyester converted products. Aromatic carboxylic acids having reduced levels of impurities may be made by further oxidizing crude products from liquid phase oxidation as described above at one or more progressively lower temperatures and oxygen levels. In addition, partial oxidation products may be recovered during crystallization and converted into the desired acid product.

Pure forms of terephthalic acid and other aromatic carboxylic acids having reduced amounts of impurities-for example, purified terephthalic acid (PTA)-have been made by catalytically hydrogenating less pure forms of the acids or so-called medium purity products in solution at elevated temperature and pressure using a noble metal catalyst. In commercial practice, liquid phase oxidation of alkyl aromatic feed materials to crude aromatic carboxylic acid, and purification of the crude product, are oftentimes conducted in continuous integrated processes in which crude product from the liquid phase oxidation is used as a starting material for the purification.

In conventional purification units, crude aromatic carboxylic acid is typically mixed with water to form a purification reaction mixture prior to its introduction to the purification reactor. The mixing occurs in a feed mix vessel that is maintained at ambient pressure in order to allow rerun aromatic carboxylic from the vessel and to enable use of a screw conveyor feed of crude aromatic carboxylic from an intermediate silo to the feed mix vessel, which requires the feed mix vessel to be pressure-equilibrated with the silo which operates at ambient pressure. When the feed mix vessel is at ambient pressure, the highest possible temperature of the water in the vessel is about 100°C. the boiling point of water at ambient pressure. The purification reaction mixture must be pre-heated prior to its introduction into the purification reactor, which typically runs at 250°C to 300°C. This heating required to raise the temperature of the purification reaction mixture from 100°C or less to at least 250°C adds to the variable cost of the integrated process for manufacturing purified aromatic carboxylic acids.

WO 2006/102137 discloses a method of recovering energy during the production of aromatic carboxylic acids by recovering heat energy from a high pressure gaseous overhead stream and recovering energy in the form of work from a high pressure off-gas.

WO 2011/100682 describes a process for the production of an aromatic carboxylic acid including the steps of introducing the crude acid into a purification reactor vessel operating under pressure, introducing hydrogen gas into the purification reactor vessel, dissolving the hydrogen gas in the solution under specific conditions, and contacting the resulting solution with a supported catalyst bed.

WO 2011/041151 provides a process for hydrogenating carboxyaryl aldehydes to hydroxyalkylaryl monocarboxylic acids by hydrogenations in the presence of a catalyst, preferably an iridium or rhodium catalyst.

US 5,723,656 relates to a process for preparing aromatic carboxylic acids in which energy is recovered from the exothermic oxidation reaction.

GB 785045 teaches the purification of terephthalic acid by heating it under pressure with water containing an oxidising agent having no oxidising effect on the terephthalic acid and cooling the resultant solution to crystallise the terephthalic acid.

GB 1047433 discloses a method of purifying aromatic dicarboxylic acids, including the steps of heating the mixture to 225 to 260 °C, followed by cooling to below 170 °C to effect crystallisation of the carboxylic acid. The resulting carboxylic acid can be further purified.

There continues to be a need to reduce the overall costs of manufacturing aromatic carboxylic acids.

### SUMMARY

The scope of the present invention is defined solely by the appended claims, and is not affected to any degree by the statements within this summary.

According to one aspect of the invention, a process for manufacturing a purified aromatic carboxylic acid is provided. A crude aromatic carboxylic acid and a solvent comprising water are mixed in a mixing zone to form a purification reaction mixture. The mixing zone is configured to operate at a pressure above ambient and the solvent is fed to the mixing zone at a pressure above ambient. The purification reaction mixture comprising the crude aromatic carboxylic acid and water is preheated in a pre-heating zone. The purification reaction mixture enters the preheating zone at a pressure above ambient and at a temperature above 100 °C. The heated purification reaction mixture then exits the pre-heating zone and enters a purification zone comprising a hydrogenation reactor and the crude aromatic carboxylic acid in the heated purification reaction mixture in the purification zone is then purified to form a purified aromatic carboxylic acid product. The purification comprises contacting at least a portion of the crude aromatic carboxylic acid with hydrogen in the presence of a catalyst in the hydrogenation reactor.

Also disclosed is a process for manufacturing a purified aromatic carboxylic acid. A purification reaction mixture comprising a crude aromatic carboxylic acid and water is preheated in a pre-heating zone. The purification reaction mixture enters the preheating zone at a temperature above 100°C. The crude aromatic carboxylic acid in purification reaction mixture is then purified to form a purified aromatic carboxylic acid product.

Other aspects of the invention will be apparent to those skilled in the art in view of the description that follows.

### BRIEF DESCRIPTION OF THE DRAWINGS

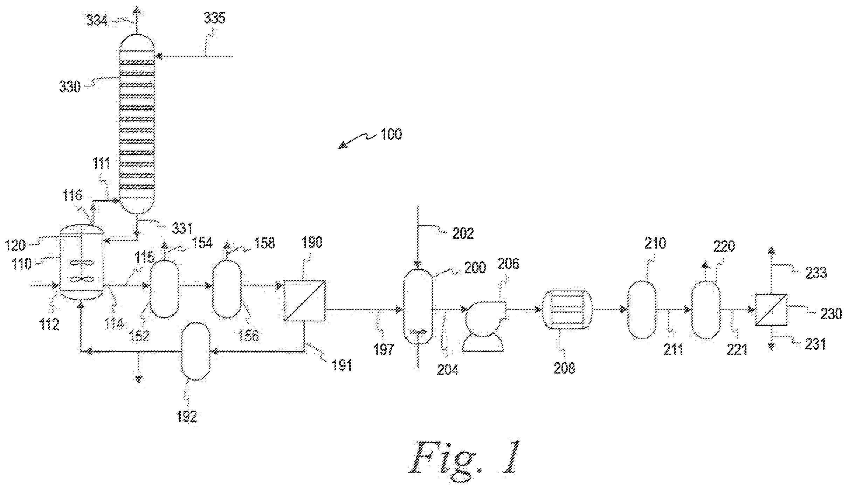

FIG. 1 shows a process flow diagram for manufacturing purified forms of aromatic carboxylic acids in accordance with the present teachings.

### DETAILED DESCRIPTION

By way of general introduction, a process for manufacturing a purified aromatic carboxylic acid in accordance with the present invention comprises: mixing crude aromatic carboxylic acid and a solvent comprising water in a mixing zone to form a purification reaction mixture, the mixing zone configured to operate at a pressure above ambient and the solvent being fed to the mixing zone at a pressure above ambient; heating the purification reaction mixture in a pre-heating zone, the purification reaction mixture comprising the crude aromatic carboxylic acid and water, the purification reaction mixture entering the pre-heating zone at a pressure above ambient and at a temperature above 100 °C; causing the heated purification reaction mixture to exit the pre-heating zone and enter a purification zone comprising a hydrogenation reactor; and purifying the crude aromatic carboxylic acid in the heated purification reaction mixture in the purification zone to form a purified aromatic carboxylic acid product, wherein purification comprises contacting at least a portion of the crude aromatic carboxylic acid with hydrogen in the presence of a catalyst in the hydrogenation reactor. In some embodiments, the purification reaction mixture also enters the pre-heating zone at temperatures above 100°C, which would not be possible if the purification reaction mixture was maintained at atmospheric pressure prior to being fed into the pre-heating zone. Accordingly, compared with prior processes, less heat is required to raise the temperature in the preheating zone to the temperature required by the purification reactor.

In some embodiments, the purification reaction mixture is introduced into pre-heating zone at a pressure that is greater than about 1.0 bar(g), in some embodiments greater than about 2.0 bar(g), in some embodiments greater than about 3.0 bar(g), in some embodiments greater than about 4.0 bar(g), in some embodiments greater than about 5.0 bar(g), and in some embodiments greater than about 6.0 bar(g). In some embodiments, the purification reaction mixture is introduced into the pre-heating zone at a temperature that is greater than about 105 °C. In some embodiments, the temperature is greater than about 115 °C, in some embodiments greater than about 1205 °C, in some embodiments greater than about 125 °C, in some embodiments greater than about 130 °C, in some embodiments greater than about 140 °C.

In some embodiments, the process further comprises forming the purification reaction mixture in a mixing by a water containing stream with a slurry comprising crude aromatic carboxylic acid. The mixing zone is maintained at a at a pressure that is greater than about 1.0 bar(g), in some embodiments greater than about 2.0 bar(g), in some embodiments greater than about 3.0 bar(g), in some embodiments greater than about 4.0 bar(g), in some embodiments greater than about 5.0 bar(g), and in some embodiments greater than about 6.0 bar(g). In some embodiments, the mixing zone is maintained at a temperature that is greater than about 105 °C. In some embodiments, the temperature is greater than about 115 °C, in some embodiments greater than about 120 °C, in some embodiments greater than about 125 °C, in some embodiments greater than about 130 °C, in some embodiments greater than about 140 °C.

Purifying the purification reaction mixture comprises contacting an aqueous solution that comprises at least a portion of the crude aromatic carboxylic acid with hydrogen in the presence of a catalyst.

In some embodiments, the process further comprises oxidizing a substituted aromatic hydrocarbon with gaseous oxygen in a liquid phase oxidation reaction mixture comprising a monocarboxylic acid solvent, water, and a catalyst composition. Purifying comprises contacting an aqueous solution that comprises at least a portion of the crude aromatic carboxylic acid with hydrogen in the presence of a catalyst.

In some embodiments, the process comprises: oxidizing paraxylene in a reaction zone to form the crude terephthalic acid, wherein the oxidizing comprises contacting the para-xylene with gaseous oxygen in a liquid phase oxidation reaction mixture that comprises acetic acid, water, and a bromine-promoted catalyst composition; crystallizing the crude terephthalic acid, transferring at least a portion of the crude terephthalic acid to a mixing zone maintained at a pressure above ambient temperature and mixing the crude terephthalic acid with a water containing stream to form a purification reaction mixture, introducing the purification reaction mixture to a pre-heating zone and heating the purification reaction mixture to at least 250°C, and purifying the crude terephthalic acid in a hydrogenation reactor by contacting the purification reaction mixture with hydrogen in the presence of a catalyst.

Additional features of the above-described processes for manufacturing purified forms of aromatic carboxylic acid in accordance with the present teachings will now be described in reference to the drawing figures.

FIG. 1 shows a process flow diagram for manufacturing purified forms of aromatic carboxylic acids in accordance with one embodiment of the present invention. As a brief introduction, the process **100** includes an oxidation reactor **110** configured for liquid phase oxidation of feedstock; a crystallization zone configured for forming crude solid product from the liquid phase oxidation reaction mixture, and comprising crystallization vessels **152** and **156**; a solid-liquid separation device **190** configured for separating crude solid product (and oxidation by-products) from liquid, a mixing zone including a purification reaction mixture make up vessel **200** configured for preparing mixtures of crude solid product in purification reaction solvent; a pre-heating zone including a heat exchanger **208** for heating the purification reaction mixture prior to its introduction into a purification zone, a purification zone including a purification reactor **210** configured for purifying the crude aromatic carboxylic acid, a crystallization zone including vessel **220** configured for forming purified solid product from the purification solution; and a solid-liquid separation device **230** configured for separating purified solid product from liquid. The integration of processes in FIG. 1 is meant to be purely representative, and various other integrated, and non-integrated configurations may likewise be used.

Liquid and gaseous streams and materials used in the process represented in FIG. 1 may be directed and transferred through suitable transfer lines, conduits, and piping constructed, for example, from materials appropriate for process use and safety. It will be understood that particular elements may be physically juxtaposed and, where appropriate, may have flexible regions, rigid regions, or a combination of both. In directing streams or compounds, intervening apparatuses and/or optional treatments may be included. By way of example, pumps, valves, manifolds, gas and liquid flow meters and distributors, sampling and sensing devices, and other equipment (e.g., for monitoring, controlling, adjusting, and/or diverting pressures, flows and other operating parameters) may be present.

Representative aromatic feedstock materials suitable for use in the oxidation reactor **110** include but are not limited to aromatic compounds (e.g., hydrocarbons) substituted at one or more positions with at least one group that is oxidizable to a carboxylic acid group. In some embodiments, the positions of the substituents correspond to the positions of the carboxylic acid groups of the aromatic carboxylic acid being prepared. In some embodiments, the oxidizable substituents include alkyl groups (e.g., methyl, ethyl, and/or isopropyl groups). In other embodiments, the oxidizable substituents include oxygen-containing groups, such as a hydroxyalkyl, formyl, aldehyde, and/or keto groups. The substituents may be the same or different. The aromatic portion of feedstock compounds may be a benzene nucleus or it may be bi- or polycyclic (e.g., a naphthalene and/or anthracene nucleus). In some embodiments, the number of oxidizable substituents on the aromatic portion of the feedstock compound is equal to the number of sites available on the aromatic portion. In other embodiments, the number of oxidizable substituents on the aromatic portion of the feedstock is fewer than all such sites (e.g., in some embodiments 1 to 4 and, in some embodiments, 2). Representative feed compounds that may be used in accordance with the present teachings-alone or in combinations-include but are not limited to toluene; ethylbenzene and other alkyl-substituted benzenes; *o*-xylene; *p-*xylene; *m*-xylene; tolualdehydes, toluic acids, alkyl benzyl alcohols, 1-formyl-4-methylbenzene, 1-hydroxymethyl-4-methylbenzene; methylacetophenone; 1,2,4-trimethylbenzene; 1-formyl-2,4-dimethyl-benzene; 1,2,4,5-tetramethyl-benzene; alkyl-, formyl-, acyl-, and hydroxylmethyl-substituted naphthalenes (e.g., 2,6-dimethylnaphthalene, 2,6-diethylnaphthalene, 2,7-dimethylnaphthalene, 2,7-diethylnaphthalene, 2-formyl-6-methylnaphthalene, 2-acyl-6-methylnaphthalene, 2-methyl-6-ethylnaphthalene, and the like); and the like; and partially oxidized derivatives of any of the foregoing; and combinations thereof. In some embodiments, the substituted aromatic compound comprises a methyl-, ethyl-, and/or isopropyl-substituted aromatic hydrocarbon. In some embodiments, the substituted aromatic compound comprises an alkyl-substituted benzene, o-xylene, *p*-xylene, *m*-xylene, or the like, or combinations thereof.

Aromatic carboxylic acids manufactured in accordance with the present teachings are not restricted and include but are not limited to mono- and polycarboxylated species having one or more aromatic rings. In some embodiments, the aromatic carboxylic acids are manufactured by reaction of gaseous and liquid reactants in a liquid phase system. In some embodiments, the aromatic carboxylic acid comprises only one aromatic ring. In other embodiments, the aromatic carboxylic acid comprises a plurality (e.g., two or more) aromatic rings that, in some embodiments, are fused (e.g., naphthalene, anthracene, etc.) and, in other embodiments, are not. In some embodiments, the aromatic carboxylic acid comprises only one carboxylic acid (e.g., -CO₂H) moiety or a salt thereof (e.g., -CO₂X, where X is a cationic species including but not limited to metal cations, ammonium ions, and the like). In other embodiments, the aromatic carboxylic acid comprises a plurality (e.g., two or more) of carboxylic acid moieties or salts thereof. Representative aromatic carboxylic acids include but are not limited to terephthalic acid, trimesic acid, trimellitic acid, phthalic acid, isophthalic acid, benzoic acid, naphthalene dicarboxylic acids, and the like, and combinations thereof. In some embodiments, the present teachings are directed to manufacture of pure forms of terephthalic acid including purified terephthalic acid (PTA) and so-called medium purity terephthalic acids.

A representative type of oxidation that may be conducted in the oxidation reactor **110** is a liquid phase oxidation that comprises contacting oxygen gas and a feed material comprising an aromatic hydrocarbon having substituents oxidizable to carboxylic acid groups in a liquid phase reaction mixture. In some embodiments, the liquid phase reaction mixture comprises a monocarboxylic acid solvent and water in the presence of a catalyst composition comprising at least one heavy metal component (e.g., Co, Mn, V, Mo, Cr, Fe, Ni, Zi, Ce, Hf, or the like, and combinations thereof) and a promoter (e.g., halogen compounds, etc.). In some embodiments, the oxidation is conducted at elevated temperature and pressure effective to maintain a liquid phase reaction mixture and form a high temperature, high-pressure vapor phase. In some embodiments, oxidation of the aromatic feed material in the liquid phase oxidation produces aromatic carboxylic acid as well as reaction by-products, such as partial or intermediate oxidation products of the aromatic feed material and/or solvent by-products. In some embodiments, the aromatic carboxylic acid comprises terephthalic acid, and the oxidizing comprises contacting para-xyiene with gaseous oxygen in a liquid phase oxidation reaction mixture that comprises acetic acid, water, and a bromine-promoted catalyst composition. The liquid-phase oxidation and associated processes may be conducted as a batch process, a continuous process, or a semi-continuous process. The oxidation may be conducted in one or more reactors.

In a representative embodiment, such as may be implemented as shown in FIG. 1, liquid feed material comprising at least about 99 wt. % substituted aromatic hydrocarbon, aqueous acetic acid solution (e.g., containing about 70 to about 95 wt. % acetic acid), soluble compounds of cobalt and manganese (e.g., such as their respective acetates) as sources of catalyst metals, bromine (e.g., hydrogen bromide) as catalyst promoter, and air may be continuously charged to oxidation reaction vessel **110** through inlets, such as inlet **112.** In some embodiments, vessel **110** is a pressurerated, continuous-stirred tank reactor.

In some embodiments, stirring may be provided by rotation of an agitator **120**, the shaft of which is driven by an external power source (not shown). Impellers mounted on the shaft and located within the liquid body are configured to provide forces for mixing liquids and dispersing gases within the liquid body, thereby avoiding settling of solids in the lower regions of the liquid body.

In some embodiments, para-xyiene is oxidized in reactor **110**, predominantly to terephthalic acid. By-products that may form in addition to terephthalic acid include but are not limited to partial and intermediate oxidation products (e.g., 4-carboxybenzaldehyde, 1,4-hydroxymethyl benzoic acid, p-toluic acid, benzoic acid, and the like, and combinations thereof). Since the oxidation reaction is exothermic, heat generated by the reaction may cause boiling of the liquid phase reaction mixture and formation of an overhead vapor phase that comprises vaporized acetic acid, water vapor, gaseous by-products from the oxidation reaction, carbon oxides, nitrogen from the air charged to the reaction, unreacted oxygen, and the like, and combinations thereof.

The overhead vapor may be removed from the reactor through vent **116** and sent in a stream **111** to high-pressure distillation column **330**. The separation zone is configured to separate water from the solvent monocarboxylic acid and return a solvent-rich liquid phase to the reactor via line **331**. A water rich gas phase is removed from the separation zone via line **334** and for further processed, for example, by recovering energy through an expander, by condensing water from the gas stream for use in the purification zone or other parts of the process, and by treatment of waste gases. Reflux is returned to the column **330** via line **335**. The reflux fluid may include condensed portions of the water rich gas stream **334** or may include fluid from other sources. Examples of further processing of the overhead gas stream and sources of reflux fluids are more fully described in US. Pat. Nos. 5,723,656, 6,137,001, 7,935,844, 7,935,845, and 8,173,834.

In some embodiments, liquid effluent comprising solid oxidation products slurried in the liquid phase reaction mixture is removed from reaction vessel **110** through slurry outlet **114** and directed in stream **115** to crystallization vessel **152**, and in turn crystallization vessel **156**, for recovery of a solid product.

In some embodiments, solid crude product may be recovered from the liquid by crystallization in one or more stages, such as in a single crystallization vessel or, as shown in FIG. 1, in a series of multiple stirred crystallization vessels. In some embodiments, the crystallization process comprises sequential reductions in temperature and pressure from earlier to later stages to increase product recovery. By way of example, as shown in FIG. 1, crystallization vessels **152** and **156** may be provided in series and in fluid communication, such that product slurry from vessel **152** may be transferred to vessel **156**. Cooling in the crystallization vessels may be accomplished by pressure release. One or more of the crystallization vessels may be vented, as at vents **154** and **158**, to remove vapor resulting from pressure let down and generation of steam from the flashed vapor to a heat exchange means (not shown).

As shown in FIG. 1, the crystallization vessel **156** is in fluid communication with a solid-liquid separation device **190**. The solid-liquid separation device **190** is configured to receive a slurry of solid product from the crystallization vessel **156**. In some embodiments, the solid-liquid separation device 190 is further configured to separate a crude solid product and by-products from the liquid. In some embodiments, the separation device **190** is a centrifuge, a rotary vacuum filter, a pressure filter, or the like, or a combination thereof. In some embodiments, the separation device **190** comprises a pressure filter configured for solvent exchange (e.g., by positive displacement under pressure of mother liquor in a filter cake with wash liquid comprising water). The oxidation mother liquor resulting from the separation may exit separation device **190** in stream **191** for transfer to mother liquor drum 192. A portion of the mother liquor and, in some embodiments, a major portion of the mother liquor, may be transferred from drum **192** to oxidation reactor **110**. In such a way, monocarboxylic acid solvent, water, catalyst, and/or oxidation reaction by-products dissolved and/or present as fine solid particles in the mother liquor may be returned to the liquid phase oxidation reaction.

As shown in FIG. 1, the stream **197** comprising heated crude solid product may be directed to a mixing zone including a reaction mixture make up vessel **200.** The crude solid product in stream **197** may be mixed and slurried in make-up vessel **200** in with a make-up solvent entering vessel **200** through line **202** to form a purification reaction mixture. The purification make-up solvent contains water. In some embodiments, the solvent line **202** connects to a holding vessel (not shown) for containing make-up solvent. In other embodiments, the solvent comprises fresh demineralized water fed from a deaerator. In other embodiments, the solvent is supplied from another part of the integrated process **100**. For example, in one embodiment, the solvent comprises the condensate obtained from an off-gas separation in column **330** or from vapors recovered from a crystallization zone Sources of purification make-up solvent are more fully described, for example, in US. Pat. Nos. 5,723,656, 6,137,001, 7,935,844, 7,935,845, and 8,173,834.

The mixing zone is configured to operate at a pressure above ambient. This pressure is maintained in vessel **200** by sealing the inlet lines **197** and **202** with pumps, filters, control valves or rotary valves (not shown) and outlet line **204** with pump **206**. In one embodiment, the pressure sealing of vessel **200** is accomplished by providing a rotary pressure filter as solid-liquid separation **190**. The rotary pressure filter pressurizes the discharged slurry exiting the device **190** and entering line **197**. Suitable rotary pressure filters are sold by BHS-Sonthofen and are disclosed for example, in US Pat Nos. 2,741,369, 7,807,060 and US Pat. App. 20050051473. Purification reaction mixture is prepared in vessel **200** is withdrawn through line **204** and transferred to pump **206**, which also acts to maintain the pressure in vessel **200**. By maintaining the vessel **200** at a pressure above ambient pressure, the purification make-up solvent **202** can enter the vessel **200** and be maintained in the vessel **200** at a pressure above ambient, and therefore may enter and be maintained at a temperature higher than would be possible than if the vessel were maintained at ambient temperature. For example, water in the purification make-up solvent could only be added to vessel at a maximum of 100°C if the vessel were maintained at one atmosphere absolute pressure. By allowing purification make-up solvent to have a higher pressure and temperature when entering the vessel **200**, the resulting purification reaction mixture formed in the vessel **200** will have a higher temperature than would be possible if the vessel **200** were not maintained at pressure.

Suitable sources of pressurized purification make-up solvent include demineralized water, steam condensate, condensate from distillation in the oxidation section, such as overhead condensed from stream **334**, and condensate from purification crystallizers such as **220**.

Purification reaction mixture exiting vessel **200** through line **204** enters a pre-heating zone. The purification reaction mixture is introduced into the pre-heating zone at a pressure above ambient, which allows the purification reaction mixture to be introduced at a higher temperature than would have been possible if non-pressurized. The pre-heating zone shown in FIG. 1 includes a pump **206** and a heat exchanger **208**. Those skilled in the art will appreciate that although only one heat exchanger is shown in FIG. 1, the pre-heating zone may include additional heat exchangers configured in series or parallel. The heat exchanger **208** raises the temperature of the purification reaction mixture to a temperature required for a purification reaction as described below. In one embodiment, the temperature is raised to at least 250°C. In one embodiment, the temperature is raised to about 290°C. Because the purification reaction mixture enters at a higher temperature than in conventional, non-pressurized systems, the energy required for heating the purification reaction mixture in the pre-heating zone is less than would be required for the conventional, non-pressurized systems.

The heated purification reaction mixture exits the pre-heating zone and enters the purification zone. The purification zone includes a purification reactor **210**. In some embodiments, purification in the purification reactor **210** comprises contacting the purification reaction mixture with hydrogen at elevated temperature and pressure in the presence of a hydrogenation catalyst. In some embodiments, the pressure ranges from about 85 to about 95 kg/cm². In some embodiments, a portion of the purification liquid reaction mixture may be continuously removed from hydrogenation reactor **210** in stream **211** and directed to a crystallization vessel **220** in a downstream crystallization zone. In crystallization vessel **220**, terephthalic acid and reduced levels of impurities may be crystallized from the reaction mixture (e.g., by reducing pressure on the liquid). The resulting slurry of purified terephthalic acid and liquid formed in vessel **220** may be directed to solid-liquid separation device **230** in stream **221**. Vapors resulting from pressure letdown in the crystallization reactor **220** may be condensed by passage to heat exchangers (not shown) for cooling. The resulting condensate liquid may be redirected to the process, for example as recycle to purification feed makeup tank (not shown), through suitable transfer lines (not shown) and/or be directed to waste water treatment (WWT). Purified terephthalic acid exits solid-liquid separation device **230** in the stream **231**. In some embodiments, at least a portion, in some embodiments all or substantially all, of the purification mother liquor may be directed in stream **233** as reflux to high- pressure distillation column **330**, as more fully described, for example, in US, Pat. Nos. 5,723,656, 8,137,001, 7,935,844, 7,935,845, and 8,173,834, In other embodiments, stream 233 may be directed to a waste water treatment facility. The solid-liquid separation device 230 may be a centrifuge, a rotary vacuum filter, a pressure filter, or the like, or a combination thereof.

The foregoing detailed description and the accompanying drawings have been provided by way of explanation and illustration.

## Claims

1. A process for manufacturing a purified aromatic carboxylic acid comprising:
mixing crude aromatic carboxylic acid and a solvent comprising water in a mixing zone to form a purification reaction mixture, the mixing zone configured to operate at a pressure above ambient and the solvent being fed to the mixing zone at a pressure above ambient;
heating the purification reaction mixture in a pre-heating zone, the purification reaction mixture comprising the crude aromatic carboxylic acid and solvent, the purification reaction mixture entering the pre-heating zone at a pressure above ambient and at a temperature above 100 °C;
causing the heated purification reaction mixture to exit the pre-heating zone and enter a purification zone comprising a hydrogenation reactor; and
purifying the crude aromatic carboxylic acid in the heated purification reaction mixture in the purification zone to form a purified aromatic carboxylic acid product, wherein purification comprises contacting at least a portion of the crude aromatic carboxylic acid with hydrogen in the presence of a catalyst in the hydrogenation reactor.

2. The process of claim 1, wherein solvent enters the mixing zone at a pressure of at least 1 bar(g).

3. The process of claim 1, wherein the solvent containing stream enters the mixing zone at a pressure of at least 2 bar(g).

4. The process of claim 1, wherein the solvent containing stream enters the mixing zone at temperature above 100°C.

5. The process of claim 1, wherein the solvent containing stream enters mixing zone at a temperature of at least 120°C.

6. The process of claim 1, wherein the mixing zone is maintained at a pressure of at least 2 bar(g).

7. The process of claim 1, wherein the purification reaction mixture enters the pre-heating zone at a pressure of at least 80 bar(g).

8. The process of claim 1, wherein the purification reaction mixture enters the pre-heating zone at a temperature of at least 120°C.

9. The process of claim 1, wherein the aromatic carboxylic acid comprises terephthalic acid.

10. The process of claim 1, further comprising:
oxidizing a substituted aromatic compound in a reaction zone to form the crude aromatic carboxylic acid.
transferring effluent from the reaction zone to a crystallization zone;
recovering at least a portion of the crude aromatic carboxylic as a solid from the crystallization zone;
transferring effluent from the crystallization zone to a solid-liquid separation device, wherein the solid-liquid separation device is configured for operation above ambient pressure;
separating solid crude aromatic carboxylic acid from liquid in a solid/liquid separation device; and
discharging the crude aromatic carboxylic acid from the solid/liquid separation device to the mixing zone.

11. The process of claim 10, wherein the solid-liquid separation device comprises a rotary pressure filter.

## Patentansprüche

1. Verfahren zur Herstellung einer gereinigten aromatischen Carbonsäure, umfassend:
Mischen der aromatischen Rohcarbonsäure und eines Lösungsmittels, umfassend Wasser in einer Mischzone zur Bildung eines Reinigungsreaktionsgemischs, wobei die Mischzone zum Betrieb bei einem Druck über dem Umgebungsdruck konfiguriert ist und wobei das Lösungsmittel bei einem Druck über dem Umgebungsdruck in die Mischzone eingetragen wird;
Erhitzen des Reinigungsreaktionsgemischs in einer Vorheizzone, wobei das Reinigungsreaktionsgemisch die aromatische Rohcarbonsäure und das Lösungsmittel umfasst, wobei das Reinigungsreaktionsgemisch in die Vorheizzone bei einem Druck über dem Umgebungsdruck und bei einer Temperatur über 100 °C eintritt;
Veranlassen des erhitzten Reinigungsreaktionsgemischs zum Verlassen der Vorheizzone und zum Eintreten in eine Reinigungszone, die einen Hydrierreaktor umfasst; und
Reinigen der aromatischen Rohcarbonsäure in dem erhitzten Reinigungsreaktionsgemisch in der Reinigungszone zur Bildung eines gereinigten aromatischen Carbonsäureprodukts, wobei die Reinigung das Inkontaktbringen von mindestens einem Anteil der aromatischen Rohcarbonsäure mit Wasserstoff bei Vorliegen eines Katalysators in dem Hydrierreaktor umfasst.

2. Verfahren nach Anspruch 1, wobei das Lösungsmittel in die Mischzone bei einem Druck von mindestens 1 bar(g) eintritt.

3. Verfahren nach Anspruch 1, wobei der Lösungsmittel enthaltende Strom in die Mischzone bei einem Druck von mindestens 2 bar(g) eintritt.

4. Verfahren nach Anspruch 1, wobei der Lösungsmittel enthaltende Strom in die Mischzone bei einer Temperatur von über 100 °C eintritt.

5. Verfahren nach Anspruch 1, wobei der das Lösungsmittel enthaltende Strom in die Mischzone bei einer Temperatur von mindestens 120 °C eintritt.

6. Verfahren nach Anspruch 1, wobei die Mischzone bei einem Druck von mindestens 2 bar(g) aufrechterhalten wird.

7. Verfahren nach Anspruch 1, wobei das Reinigungsreaktionsgemisch in die Vorheizzone bei einem Druck von mindestens 80 bar(g) eintritt.

8. Verfahren nach Anspruch 1, wobei das Reinigungsreaktionsgemisch in die Vorheizzone bei einer Temperatur von mindestens 120 °C einritt.

9. Verfahren nach Anspruch 1, wobei die aromatische Carbonsäure Terephthalsäure umfasst.

10. Verfahren nach Anspruch 1, weiter umfassend:
Oxidieren einer substituierten aromatischen Verbindung in einer Reaktionszone zur Bildung der aromatischen Rohcarbonsäure;
Überführen des Ablaufs aus der Reaktionszone an eine Kristallisationszone;
Gewinnen mindestens eines Anteils der aromatischen Rohcarbonsäure als einen Feststoff aus der Kristallisationszone;
Überführen des Ablaufs aus der Kristallisationszone in eine Fest-Flüssig-Trennvorrichtung, wobei die Fest-Flüssig-Trennvorrichtung zum Betrieb über dem Umgebungsdruck konfiguriert ist;
Trennen der festen aromatischen Rohcarbonsäure aus der Flüssigkeit in einer Fest/Flüssig-Trennvorrichtung; und
Austrag der aromatischen Rohcarbonsäure aus der Fest/Flüssig-Trennvorrichtung in die Mischzone.

11. Verfahren nach Anspruch 10, wobei die Fest-Flüssig-Trennvorrichtung ein Drehdruckfilter umfasst.

## Revendications

1. Procédé pour la fabrication d'un acide carboxylique aromatique purifié comprenant :
le mélange d'un acide carboxylique aromatique brut et d'un solvant comprenant de l'eau dans une zone de mélange pour former un mélange réactionnel de purification, la zone de mélange étant configurée pour fonctionner à une pression au-dessus de la pression ambiante et le solvant étant introduit dans la zone de mélange à une pression au-dessus de la pression ambiante ;
le chauffage du mélange réactionnel de purification dans une zone de préchauffage, le mélange réactionnel de purification comprenant l'acide carboxylique aromatique brut et le solvant, le mélange réactionnel de purification entrant dans la zone de préchauffage à une pression au-dessus de la pression ambiante et à une température au-dessus de 100 °C ;
le fait d'amener le mélange réactionnel de purification chauffé à sortir de la zone de préchauffage et à entrer dans une zone de purification comprenant un réacteur d'hydrogénation ; et
la purification de l'acide carboxylique aromatique brut dans le mélange réactionnel de purification chauffé dans la zone de purification pour former un produit acide carboxylique aromatique purifié, où la purification comprend la mise en contact d'au moins une partie de l'acide carboxylique aromatique brut avec de l'hydrogène en présence d'un catalyseur dans le réacteur d'hydrogénation.

2. Procédé selon la revendication 1, où le solvant entre dans la zone de mélange à une pression d'au moins 1 bar(g).

3. Procédé selon la revendication 1, où le courant contenant le solvant entre dans la zone de mélange à une pression d'au moins 2 bar(g).

4. Procédé selon la revendication 1, où le courant contenant le solvant entre dans la zone de mélange à une température au-dessus de 100 °C.

5. Procédé selon la revendication 1, où le courant contenant le solvant entre dans la zone de mélange à une température d'au moins 120 °C.

6. Procédé selon la revendication 1, où la zone de mélange est maintenue à une pression d'au moins 2 bar(g).

7. Procédé selon la revendication 1, où le mélange réactionnel de purification entre dans la zone de préchauffage à une pression d'au moins 80 bar(g).

8. Procédé selon la revendication 1, où le mélange réactionnel de purification entre dans la zone de préchauffage à une température d'au moins 120 °C.

9. Procédé selon la revendication 1, où l'acide carboxylique aromatique comprend de l'acide téréphtalique.

10. Procédé selon la revendication 1, comprenant en outre :
l'oxydation d'un composé aromatique substitué dans une zone de réaction pour former l'acide carboxylique aromatique brut ;
le transfert de l'effluent de la zone de réaction vers une zone de cristallisation ;
la récupération d'au moins une partie de l'acide carboxylique aromatique brut sous forme de solide de la zone de cristallisation ;
le transfert de l'effluent de la zone de cristallisation vers un dispositif de séparation solide-liquide, où le dispositif de séparation solide-liquide est configuré pour un fonctionnement au-dessus de la pression ambiante ;
la séparation de l'acide carboxylique aromatique brut solide du liquide dans un dispositif de séparation solide/liquide ; et
l'évacuation de l'acide carboxylique aromatique brut du dispositif de séparation solide/liquide vers la zone de mélange.

11. Procédé selon la revendication 10, où le dispositif de séparation solide-liquide comprend un filtre rotatif sous pression.
